# EUROPÄISCHE PATENTANMELDUNG

(11) **EP 1 925 256 A1**
(43) Veröffentlichungstag der Anmeldung: **28.05.2008**
(21) Anmeldenummer: 06024421.7
(22) Anmeldetag: 24.11.2006
(51) Int. Cl.: A61B 6/12

(54) **Verfahren und Vorrichtung zur Registrierung einer anatomischen Struktur mit Markern**

(71) Anmelder: BrainLAB AG, 85622 Feldkirchen (DE)
(72) Erfinder: Drumm, Peter, 81543 München (DE); Hepke, Markus, 80538 München (DE)
(74) Vertreter: Schwabe - Sandmair - Marx

(57) **Zusammenfassung**

Die Erfindung betrifft ein Verfahren zum Registrieren einer anatomischen Struktur (7) mit mindestens einem Marker (5), wobei ein dreidimensionales Modell der Struktur (7) mit einem bildgebenden Verfahren durch Aufnahme der Struktur (7) erzeugt wird, mindestens zwei zweidimensionale Aufnahmen der Struktur (7) aus unterschiedlichen Winkeln gemacht werden und durch ein Matching-Verfahren anhand der Positionen des oder der Marker (5) in den mindestens zwei zweidimensionalen Abbildungen die Position und Lage des dreidimensionalen Modells im Raum oder so ermittelt wird, dass das dreidimensionale Modell der Struktur (7) mit der Struktur (7) übereinstimmt.

## Beschreibung

Die vorliegende Erfindung betrifft ein Verfahren und eine Vorrichtung zum Registrieren einer Struktur, wie zum Beispiel einer anatomischen Struktur, beispielsweise eines Beckens, mit Markern, wie zum Beispiel kugelförmigen Tantal-Markern, welche beispielsweise vor der Durchführung des Verfahrens in die anatomische Struktur oder einen Knochen eingebracht wurden.

Vor dem Durchführen eines chirurgischen Eingriffes ist es häufig erforderlich, eine anatomische Struktur zu registrieren, d.h. die Lage der Struktur oder eines Teilbereiches im Raum, wie zum Beispiel die dreidimensionale Position von charakteristischen Punkten oder Landmarken an oder auf der Struktur, zu bestimmen. Diese charakteristischen Punkte oder Landmarken werden verwendet, um zum Beispiel bei einer Hüftoperation die Lage von charakteristischen Ebenen im Raum zu bestimmen, durch welche die Position einer einzusetzenden Hüftgelenkspfanne bestimmt werden kann.

Werden bei einer Hüftoperation mit einem C-Bogen (C-arm) Aufnahmen des Beckens gemacht, so ist es auf Grund der verschiedenen Gewebearten und der möglicherweise zusätzlich vorhandenen Operationsinstrumente schwierig oder unmöglich, eine charakteristische oder eindeutige Kontur des Beckens zu erkennen. Weiterhin kann es schwierig sein, eine Kontur oder einen charakteristischen Punkt zu detektieren, da die anatomischen Strukturen bei verschiedenen Patienten unterschiedlich sind, so dass es bei der Registrierung der anatomischen Struktur zu Ungenauigkeiten und infolge dessen zu Problemen bei oder nach dem chirurgischen Eingriff kommen kann.

Aus der WO 2005/084541 A1 sind ein Verfahren und eine Vorrichtung zum Registrieren eines Beckens bekannt, wobei ein Pointer mit trackbaren Markern, welche von einem Tracking-System detektiert werden können, zum Akquirieren von Punkten verwendet wird.

Es ist eine Aufgabe der vorliegenden Erfindung ein Verfahren und eine Vorrichtung vorzuschlagen, welche ein einfaches, schnelles und sicheres Registrieren einer Struktur ermöglichen.

Diese Aufgabe wird durch das Verfahren und die Vorrichtung wie in den unabhängigen Ansprüchen definiert bestimmt. Vorteilhafte Ausführungsformen ergeben sich aus den abhängigen Ansprüchen.

Bei dem erfindungsgemäßen Verfahren zum Registrieren einer Struktur, wie zum Beispiel einer anatomischen Struktur, kann eine in der Struktur selbst liegende oder in einem dreidimensionalen Modell der Struktur liegende Kontur und/oder eine oder mehrere Landmarken unter Verwendung mindestens zweier zweidimensionaler Aufnahmen eines bildgebenden Verfahrens detektiert und die Struktur so zum Beispiel registriert werden, indem ein oder mehrere zum Beispiel radioopake Marker an oder in der Struktur befestigt oder darin eingebracht sind, wobei die Marker durch das jeweils verwendete bildgebende Verfahren detektiert oder erkannt werden können. Beispielsweise können der oder die Marker in einen Knochen oder die Knochenoberfläche einer Struktur, wie zum Beispiel eines Beckens, bevorzugt an einem charakteristischen Punkt oder einer charakteristischen Linie eingebracht oder eingesetzt werden, so dass es erfindungsgemäß nicht mehr erforderlich ist, den charakteristischen Punkt oder die Landmarke zu detektieren, wenn der zugehörige Marker oder eine Markergruppe aufgenommen werden. Die Positionen der Marker in den mindestens zwei zweidimensionalen Aufnahmen der Struktur können rückprojeziert werden und die sich bei der Rückprojektion ergebenden Schnittpunkte können mit der zum Beispiel durch ein CT-Verfahren dreidimensional erfassten Struktur verglichen werden, um die jeweiligen Markerpositionen in Übereinstimmung zu bringen und die Struktur so zu registrieren.

Es ist jedoch nicht zwingend nötig ein Modell bzw. präoperative Bilddatensätze aufzunehmen. Die Markerpositionen allein können schon während der Operation charakteristische und zum Beispiel die Lage oder Ausrichtung des Objekts definierende Ebenen, wie zum Beispiel die Midsagittalebene oder vordere Beckenebene, Geraden oder Punkte, wie zum Beispiel Spinapunkte, in registrieren 2D Bildern definieren, die ausreichend für die Operation sein können. Zusätzlich kann auch ein Bilddatensatz, wie zum Beispiel ein CT, mit den Markerpositionen, die zuvor eingebracht wurden, registriert werden.

Insbesondere wenn die zu registrierende Struktur mit bereits angebrachten oder eingesetzten Markern vor der Durchführung des Verfahrens, zum Beispiel durch einen CT-Scan, aufgenommen wurde, um ein dreidimensionales Computermodell der Struktur mit darin eingesetzten Markern zu generieren, kann durch ein bekanntes Matching-Verfahren schnell und einfach ermittelt werden, wie die Zuordnung der auf den mindestens zwei zweidimensionalen Aufnahmen der Struktur sichtbaren Marker zu dem dreidimensionalen Computermodell ist. Hieraus kann dann zum Beispiel die Position eines oder mehrerer charakteristischer Punkte oder Landmarken, welche ein aus der dreidimensionalen Aufnahme der Struktur bekanntes Lageverhältnis zu den Markern haben, ermittelt werden.

Erfindungsgemäß ist es somit möglich, bekannte Geometrien, Linien oder Punkte einer Struktur mittels zum Beispiel zweier Fluoroskopie-Bilder zu bestimmen, ohne dass die Notwendigkeit besteht, diese charakteristischen Punkte oder Landmarken zum Beispiel mit einem Pointer an der Struktur selbst anzufahren.

Soll zum Beispiel ein Becken registriert werden, so kann die Beckenkontur in intraoperativ gewonnenen 2D Bildern des Patienten detektiert werden und es können zum Beispiel daraus als weitere Information charakteristische Ebenen berechnet werden. Die 2D Bilder können über eine Referenz am Patienten, wie zum Beispiel einen an dem Pattienten befestigten Referenzstern, und das Fluoro Kit am C-Bogen schon automatisch registriert werden. Für die Ausrichtung der Implantatkomponenten können auch weitere Informationen, wie zum Beispiel Körperebenen, verwendet werden, die aus der Beckenkontur in den registrierten Bildern gewonnen werden.

Wird zur Erzeugung der mindestens zwei zweidimensionalen Aufnahmen der Struktur ein kalibriertes Aufnahmeverfahren oder eine kalibrierte Aufnahmevorrichtung, wie zum Beispiel ein kalibrierter C-Bogen verwendet, an welchem zum Beispiel mehrere durch ein Navigationssystem erkennbare Marker angebracht sind, so können anhand der aus mindestens zwei unterschiedlichen Blickrichtungen gemachten registrierten Aufnahmen oder Fluoroskopie-Bildern, in welchen die mit der Struktur verbundenen Marker sichtbar sind, die dreidimensionalen Positionen der Marker aus den zweidimensionalen Markerpositionen in den Aufnahmen oder Bildern zum Beispiel durch Rückprojektion bestimmt oder berechnet werden. Somit können die dreidimensionalen Positionen der Marker im Raum ermittelt werden und aus den so ermittelten Marker-Positionen kann nach einem Matching-Verfahren unter Verwendung einer vorher durchgeführten dreidimensionalen Aufnahme der Struktur die Position und Ausrichtung der dreidimensionalen Struktur im Raum bestimmt werden, wodurch die Struktur automatisch registriert werden kann. Durch das erfindungsgemäße Verfahren kann somit eine Struktur einfacher registriert werden, da nur die Position eines oder mehrerer Marker ermittelt werden müssen und es nicht erforderlich ist, eine sich zum Beispiel in Abhängigkeit von der Richtung der jeweiligen zweidimensionalen Aufnahme verändernde Kontur zu detektieren und auf ein dreidimensionales Computermodell zu matchen.

Vorteilhaft werden die mindestens zwei zweidimensionalen Aufnahmen der Struktur aus unterschiedlichen Richtungen mit einer kalibrierten Röntgen-Quelle und einem zugehörigen kalibrierten Detektor, wie zum Beispiel einem kalibrierten C-Bogen, gemacht, wobei die zwei Transformations-Matritzen der jeweiligen Röntgenaufnahmen bekannt sind. Die Koordinaten der Bilder und auch die Information in den Bildern können durch diese zwei Matritzen in ein Patientenkoordinatensystem transformiert werden. In diesem Patientenkoordinatensystem kann ein Matching der transformierten Information mit einem dreidimensionalen Computermodell, welches zum Beispiel durch eine vorher durchgeführte CT- oder MR-Aufnahme erhalten wurde, durchgeführt werden.

Das erfindungsgemäße Verfahren kann zum Beispiel auch mit nur einem Marker durchgeführt werden, wenn zum Beispiel eine oder mehr Landmarken durch andere Verfahren, zum Beispiel mit einem Pointer, akquiriert werden.

Vorzugsweise werden Objekte aus Tantal, wie zum Beispiel Tantal-Kugeln bevorzugt mit einem Durchmesser von kleiner als 1 mm als Marker verwendet, welche in den Knochen oder die Knochenoberfläche eingebracht werden. Die Verwendung von Tantal-Markern, welche in einen Knochen eingebracht werden und mittels eines Pointers lokalisiert werden, ist aus der WO 97/32522 bekannt. Ebenso können auch zum Beispiel Quader oder sonstige kantige Objekte, die eine gute Verankerung im Knochen sicherstellen, als Marker verwendet werden. Denkbar ist auch ein Marker, der durch die Rotation oder eine andere Bewegung im Knochen fixiert wird.

Die Erfindung bezieht sich auch auf ein Computerprogramm welches, wenn es in einen Computer geladen ist oder auf einem Computer läuft, ein wie oben beschriebenes Verfahren ausführt. Des Weiteren bezieht sich die Erfindung auf ein Programmspeichermedium oder Computerprogrammprodukt mit einem solchen Programm.

Eine erfindungsgemäße Vorrichtung zum Registrieren einer anatomischen Struktur weist eine Aufnahmeeinheit zur Erzeugung mindestens zweier zweidimensionaler Abbildungen der Struktur durch Aufnahmen aus unterschiedlichen Richtungen, wie zum Beispiel einen C-Bogen, auf. Der C-Bogen ist mit einer Recheneinheit verbunden, welche in einer Datenbank ein dreidimensionales Modell der zu registrierenden Struktur gespeichert hat. Dieses dreidimensionale Modell der Struktur kann zum Beispiel von einem mit der Recheneinheit verbundenen CT-Scanner erzeugt worden sein. Weiterhin weist die erfindungsgemäße Vorrichtung ein Navigationssystem auf, mit welchem die Position von zum Beispiel sichtbares oder Infrarot-Licht reflektierenden oder emittierenden Markern erfasst werden kann. An der Aufnahmevorrichtung, wie zum Beispiel dem C-Bogen, sind ein oder mehrere Marker angebracht, so dass kalibrierte Aufnahmen, wie zum Beispiel kalibrierte Fluoro-Schüsse, erzeugt und an die Recheneinheit übermittelt werden können, so dass die Recheneinheit aus den kalibrierten Aufnahmen der Struktur und dem dreidimensionalen Modell der Struktur die Struktur mit dem oben beschriebenen Verfahren registrieren kann und somit die Position und Ausrichtung oder Orientierung des dreidimensionalen Modells der tatsächlich vorliegenden und von der Aufnahmevorrichtung aus unterschiedlichen Richtungen aufgenommenen Struktur zuordnet, so dass im Idealfall das Modell und die tatsächlich vorliegende Struktur vollständig übereinstimmen.

Die Erfindung wird nachfolgend anhand von Ausführungsbeispielen beschrieben. Es zeigen:
- Figur 1: ein Injektionsinstrument zum Einbringen von Markern;
- Figur 2: eine Querschnittsansicht eines Knochens mit der Injektionsvorrichtung gemäß Figur 1;
- Figur 3: eine Röntgenaufnahme eines Femurs mit eingesetzten Tantal-Markern;
- Figuren 4A und 4B: die Abbildung einer Tantal-Kugel im Spina iliaca; und
- Figur 5: ein Ablaufdiagramm des erfindungsgemäßen Verfahrens.

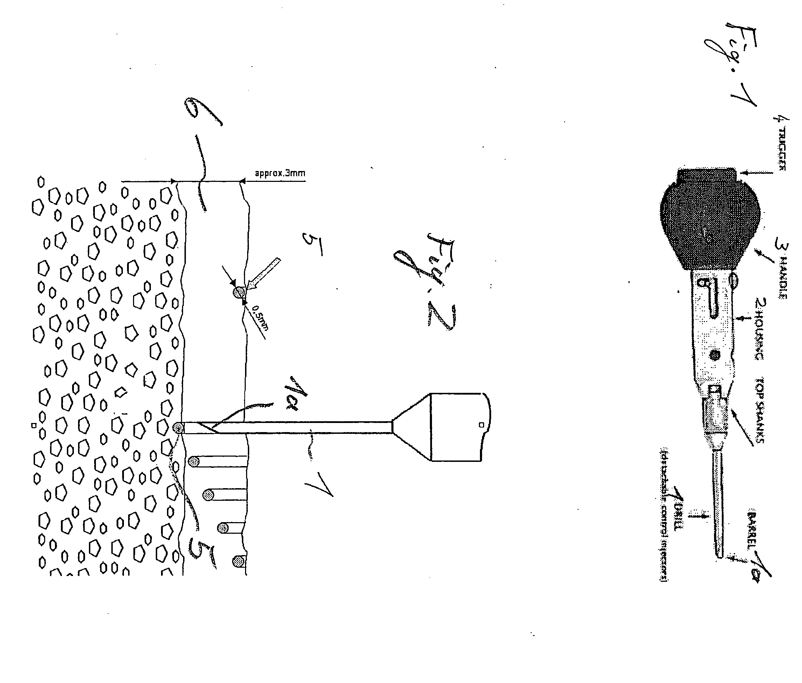

Figur 1 zeigt eine Injektionsvorrichtung zum Einbringen von kugelförmigen Tantal-Markern 5 in Knochen 6 mit einem hohlen Bohrer 1, welcher an seiner Spitze eine Öffnung 1a aufweist, durch welche ein Tantal-Kügelchen 5 abgegeben werden kann, wenn mit dem Bohrer 1 eine Vertiefung oder ein Loch in einen Knochen 6 mit der Tiefe von zum Beispiel 3 mm gebohrt wurde. Der Bohrer 1 ist abnehmbar an einem Gehäuse 2 befestigt, welches einen Griff 3 und einen Auslöseknopf 4 aufweist, wobei die Drehbewegung des Bohrers 1 entweder durch manuelle Betätigung des Griffes 3 oder durch ein in dem Gehäuse 2 des Instruments vorgesehenen Motor bewirkt wird. Bei der gewünschten Eindringtiefe kann der Knopf 4 betätigt werden, um eine Tantal-Kugel 5 in den Knochen 6 einzubringen und zum Beispiel einzuschießen.

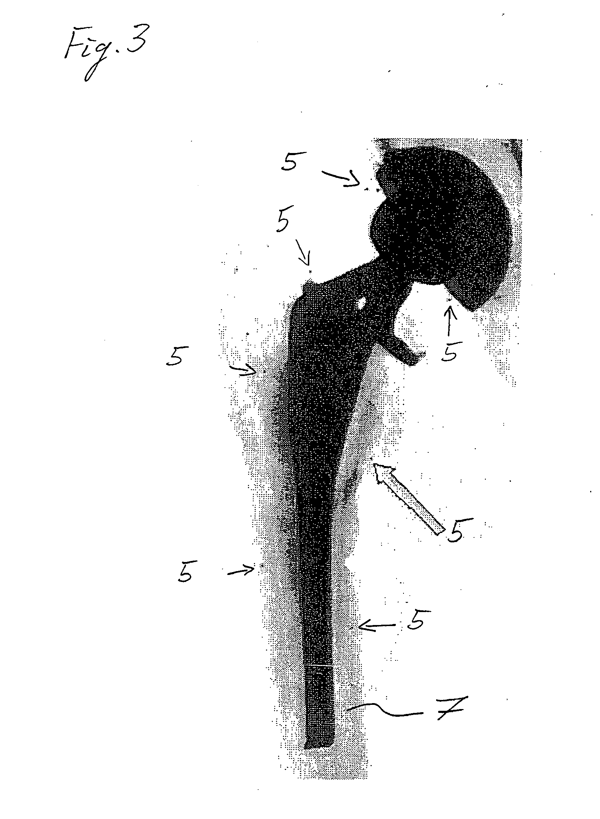

Figur 2 zeigt in Querschnittsansicht den Bohrer 1, welcher die etwa 3 mm dicke kompakte Knochenschicht durchdringt, um einen kugelförmigen Tantal-Marker 5 mit einem Durchmesser von 0,5 mm in den Knochen 6 einzusetzen. Ein so eingebrachter Marker 5 sitzt durch Presspassung sicher in dem Knochen 6.

Werden, wie in Figur 2 gezeigt, mehrere Tantal-Marker 5 in den Knochen 6 eingebracht, so können diese Marker 5 leicht in einem zweidimensionalen Röntgenbild der Knochenstruktur erkannt werden, wie in der Röntgenaufnahme des Femurs 7 in Figur 3 gezeigt. Die Koordinaten der Massenmittelpunkte der kugelförmigen Marker 5 können berechnet werden und können, wenn mehrere kalibrierte Röntgenaufnahmen erzeugt wurden, in das PatientenKoordinatensystem transformiert werden. Sind mehrere Marker 5 vorhanden oder werden zusätzlich durch bekannte Verfahren, wie zum Beispiel Punktakquisition mit einem Pointer, andere charakteristische Punkte oder Landmarken der Struktur akquiriert, kann die Registrierung der anatomischen Struktur wie oben beschrieben vorgenommen werden.

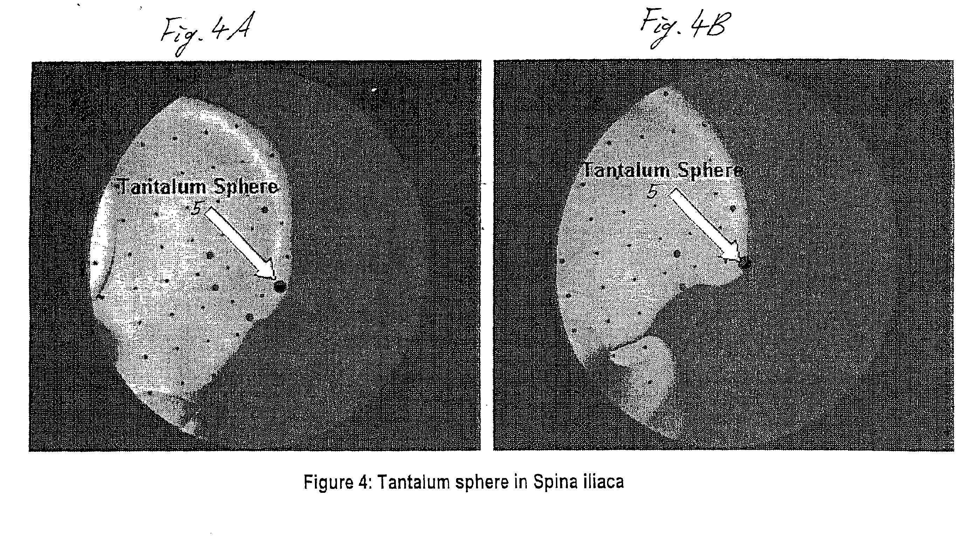

Die Figuren 4A und 4B zeigen in vergrößerter Darstellung die Abbildung eines Tantal-Markers 5 in der Spina iliaca aus zwei unterschiedlichen Richtungen, wobei aus den beiden in den Figuren 4A und 4B gezeigten kalibrierten Aufnahmen durch Rückprojektion der in den beiden zweidimensionalen Aufnahmen ermittelten Markerposition die Position des Tantal-Markers 5 im dreidimensionalen Raum bestimmt werden kann. Da bei einem chirurgischen Eingriff an der Hüfte in lateraler Position des Patienten beide Seiten des Patienten zum Beispiel mit einem mechanischen Pointer nicht erreicht werden können, kann aus den beiden in den Figuren 4A und 4B gezeigten Aufnahmen die Position der Spina iliaca im Patientenkoordinatensystem auch an der nicht frei zugänglichen gesunden Seite, auf welcher der Patient liegt, ermittelt werden, so dass aus der so ermittelten Landmarke zusammen mit einer weiteren zum Beispiel ebenfalls durch Tantal-Marker 5 markierte oder mittels eines Pointers akquirierte Landmarken eine Registrierung des Beckens vorgenommen werden kann.

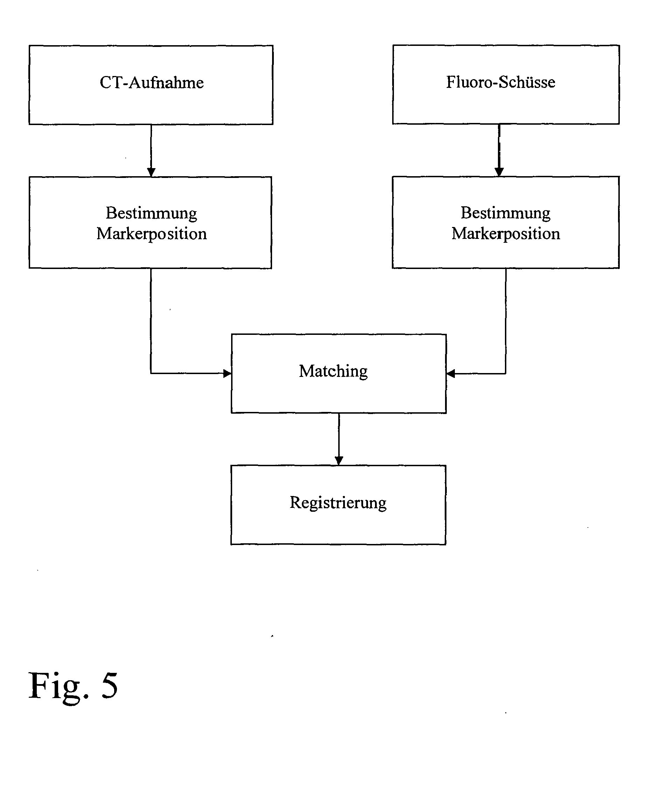

Figur 5 zeigt ein Ablaufdiagramm des erfindungsgemäßen Verfahrens, wobei ein dreidimensionales Modell der Struktur, wie zum Beispiel einer Hüfte, mittels einer CT-Aufnahme erzeugt wird und in dem dreidimensionalen Modell die Positionen der eingesetzten Marker bestimmt werden. Von der anatomischen Struktur oder dem Becken werden aus zwei unterschiedlichen Richtungen kalibrierte Fluoro-Schüsse gemacht, um zwei zweidimensionale Röntgenaufnahmen der Struktur zu haben, wobei wiederum die Positionen der in den Röntgenaufnahmen sichtbaren Marker ermittelt wird. Anschließend wird ein bekanntes Matching-Verfahren durchgeführt, um das aus der CT-Aufnahme ermittelte dreidimensionale Modell unter Verwendung der durch die Fluoro-Schüsse ermittelten Lageinformation so im dreidimensionalen Raum oder Patientenkoordinatensystem auszurichten, dass das dreidimensionale Modell der Struktur mit der tatsächlichen Struktur übereinstimmt, wodurch die Struktur oder das Becken registriert ist.

## Patentansprüche

1. Verfahren zum Registrieren einer anatomischen Struktur (7) mit mindestens einem Marker (5), wobei ein dreidimensionales Modell der Struktur (7) mit einem bildgebenden Verfahren durch Aufnahme der Struktur (7) erzeugt wird, mindestens zwei zweidimensionale Aufnahmen der Struktur (7) aus unterschiedlichen Winkeln gemacht werden und durch ein Matching-Verfahren anhand der Positionen des oder der Marker (5) in den mindestens zwei zweidimensionalen Abbildungen die Position und Lage des dreidimensionalen Modells im Raum oder einem Patientenkoordinatensystem so ermittelt wird, dass das dreidimensionale Modell der Struktur (7) mit der Struktur (7) übereinstimmt.

2. Verfahren nach Anspruch 1, wobei bei dem Matching-Verfahren in jeder der zweidimensionalen Aufnahmen der Struktur (7) die Position des mindestens einen Markers (5) ermittelt wird und durch Rückprojektion der in den zweidimensionalen Aufnahmen ermittelten Marker-Positionen die dreidimensionale Position des mindestens einen Markers (5) im Raum oder einem Patientenkoordinatensystem bestimmt wird.

3. Verfahren nach einem der vorhergehenden Ansprüche, wobei bei dem Matching-Verfahren die Position der Marker (5) in der dreidimensionalen Aufnahme ermittelt wird und das dreidimensionale Modell der Struktur (7) so orientiert wird, dass an den durch Rückprojektionen der Positionen der in den zweidimensionalen Aufnahmen erkennbaren Marker ermittelten Stellen jeweils ein Marker (5) des dreidimensionalen Modelles der Struktur (7) liegt.

4. Verfahren nach einem der vorhergehenden Ansprüche, wobei die mindestens zwei zweidimensionalen Aufnahmen kalibriert sind.

5. Verfahren nach einem der vorhergehenden Ansprüche, wobei die dreidimensionale Aufnahme der Struktur (7) eine Computertomographie (CT)-oder eine Kernspinresonanz (MR)-Aufnahme ist.

6. Verfahren nach einem der vorhergehenden Ansprüche, wobei als Marker (5) kugelförmige, quaderförmige oder kantige Objekte verwendet werden.

7. Verfahren nach einem der vorhergehenden Ansprüche, wobei die Marker (5) aus radioopakem Material oder Tantal sind.

8. Verfahren zum Definieren von Punkten, Geraden oder Ebenen in registrierten 2D Bildern eines Objektes, wobei an Hand von in das Objekt eingebrachten Markern (5), deren Positionen relativ zum Objekt bekannt sind, charakteristische Punkte, Geraden oder Ebenen des Objekts errechnet werden.

9. Computerprogramm welches, wenn es auf einem Computer läuft oder in einen Computer geladen ist, das Verfahren nach einem der vorhergehenden Ansprüche durchführt.

10. Programmspeichermedium oder Computerprogrammprodukt mit dem Verfahren nach dem vorhergehenden Anspruch.

11. Vorrichtung zum Registrieren einer Struktur mit einer Aufnahmeeinheit zur Erzeugung mindestens zweier zweidimensionaler Abbildungen der Struktur durch Aufnahmen aus unterschiedlichen Richtungen und einer Recheneinheit, welche mit der Aufnahmeeinheit verbunden ist und welche in einer Datenbank ein dreidimensionales Modell der zu registrierenden Struktur gespeichert hat und welche das Verfahren nach einem der vorhergehenden Ansprüche durchführen oder steuern kann.

12. Vorrichtung nach dem vorhergehenden Anspruch, wobei an der Aufnahmeeinheit ein oder mehrere Marker angebracht sind, um kalibrierte Aufnahmen erzeugen zu können.

13. Vorrichtung nach einem der zwei vorhergehenden Ansprüche mit einem Navigationssystem, mit welchem die Position von Licht reflektierenden oder emittierenden mit der Struktur verbundenen Markern erfasst werden kann.
